(19) Europäisches Patentamt
European Patent Office
Office européen des brevets

(11) **EP 2 966 112 A1**

(12) **EUROPEAN PATENT APPLICATION**
published in accordance with Art. 153(4) EPC

(43) Date of publication:
**13.01.2016 Bulletin 2016/02**

(51) Int Cl.:
*C08G 77/24* (2006.01)          *B82Y 30/00* (2011.01)
*B82Y 40/00* (2011.01)          *C07C 31/38* (2006.01)
*C07F 7/04* (2006.01)           *C08G 77/18* (2006.01)

(21) Application number: **14759466.7**

(22) Date of filing: **06.03.2014**

(86) International application number:
**PCT/JP2014/055818**

(87) International publication number:
**WO 2014/136893 (12.09.2014 Gazette 2014/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **06.03.2013   JP 2013044081
06.03.2013   JP 2013044082
06.03.2013   JP 2013044083**

(71) Applicants:
• **Unimatec Co., Ltd.
Tokyo 105-0012 (JP)**

• **Hirosaki University
Hirosaki-shi, Aomori 036-8560 (JP)**

(72) Inventors:
• **SATO Katsuyuki
Kitaibaraki-shi
Ibaraki 319-1544 (JP)**
• **SAWADA Hideo
Hirosaki-shi
Aomori 036-8560 (JP)**

(74) Representative: **Beckmann, Claus
Kraus & Weisert
Patentanwälte PartGmbB
Thomas-Wimmer-Ring 15
80539 München (DE)**

(54) **FLUORINE-CONTAINING NANO-SILICA COMPOSITE PARTICLES AND PREPARATION METHOD THEREFOR**

(57)    Fluorine-containing nano-silica composite particles comprising a condensate of a fluorine-containing alcohol represented by the general formula:

$$R_F\text{-A-OH} \qquad [I]$$

wherein $R_F$ is a perfluoroalkyl group or a polyfluoroalkyl group in which some of the fluorine atoms of the perfluoroalkyl group are replaced by hydrogen atoms, and A is an alkylene group having 1 to 6 carbon atoms; and an alkoxysilane with nano-silica particles,
or fluorine-containing nano-silica composite particles comprising a condensate of a fluorine-containing alcohol represented by the general formula:

$$R_F'\text{-A-OH} \qquad [Ia]$$

or the general formula:

$$\text{HO-A-}R_F''\text{-A-OH} \qquad [Ib]$$

wherein $R_F'$ is a linear or branched perfluoroalkyl group containing an O, S, or N atom, $R_F''$ is a linear or branched perfluoroalkylene group containing an O, S, or N atom, and A is an alkylene group having 1 to 6 carbon atoms; and an alkoxysilane with nano-silica particles.

EP 2 966 112 A1

**Description**

TECHNICAL FIELD

The present invention relates to fluorine-containing nano-silica composite particles and a method for producing the same. More particularly, the present invention relates to fluorine-containing nano-silica composite particles using a fluorine-containing alcohol, and a method for producing the same.

BACKGROUND ART

Patent Document 1 discloses a liquid, fluorine-containing and single-component composition for the permanent oil- and water-repellent surface treatment of porous and nonporous substrates, wherein the composition comprises a suitable stabilizing component and a hydrophilic silane component in combination, and has excellent storage stability, and hydrophobic, oleophobic and dust proof properties.

However, in the preparation of a surface treating agent for mineral and non-mineral substrates, a highly toxic isocyanate compound is used to introduce a silyl group into a fluorine compound. Therefore, its implementation requires the regulation of the production environment. Moreover, perfluorooctanoic acid and a fluorine-containing alcohol containing a perfluoroalkyl group having 8 or more carbon atoms, which is a precursor of perfluorooctanoic acid, are used, although less use of them is currently desired in terms of the current state of the environment.

PRIOR ART DOCUMENTS

PATENT DOCUMENTS

   Patent Document 1: JP-A-2011-511113

   Patent Document 2: JP-B-4674604

   Patent Document 3: WO 2007/080949 A1

   Patent Document 4: JP-A-2008-38015

   Patent Document 5: USP 3,574,770

OUTLINE OF THE INVENTION

PROBLEM TO BE SOLVED BY THE INVENTION

An object of the present invention is to provide fluorine-containing nano-silica composite particles having excellent water- and oil-repellency, and using a fluorine-containing alcohol which does not produce perfluorooctanoic acid and the like, even when released into the environment, in the case of a perfluoroalkyl group having less than 8 carbon atoms, and to provide a method for producing the same.

MEANS FOR SOLVING THE PROBLEM

The present invention provides fluorine-containing nano-silica composite particles comprising a condensate of a fluorine-containing alcohol represented by the general formula:

$$R_F\text{-A-OH} \qquad [I]$$

wherein $R_F$ is a perfluoroalkyl group or a polyfluoroalkyl group in which some of the fluorine atoms of the perfluoroalkyl group are replaced by hydrogen atoms, and A is an alkylene group having 1 to 6 carbon atoms; and an alkoxysilane with nano-silica particles.

The fluorine-containing nano-silica composite particles are produced by a method comprising subjecting the above fluorine-containing alcohol [I] and an alkoxysilane to a condensation reaction in the presence of nano-silica particles using an alkaline or acidic catalyst. The obtained fluorine-containing nano-silica composite particles are used as an active ingredient of surface treating agents, such as water- and oil-repellents.

Moreover, the present invention provides fluorine-containing nano-silica composite particles comprising a condensate of a fluorine-containing alcohol represented by the general formula:

$$R_F'\text{-A-OH} \qquad [Ia]$$

or the general formula:

$$HO\text{-A-}R_F''\text{-A-OH} \qquad [Ib]$$

wherein $R_F'$ is a linear or branched perfluoroalkyl group containing an O, S, or N atom, $R_F''$ is a linear or branched perfluoroalkylene group containing an O, S, or N atom, and A is an alkylene group having 1 to 6 carbon atoms; and an alkoxysilane with nano-silica particles.

The fluorine-containing nano-silica composite particles are produced by a method comprising subjecting the above fluorine-containing alcohol [Ia] or [Ib] and an alkoxysilane to a condensation reaction in the presence of nano-silica particles using an alkaline or acidic catalyst. The obtained fluorine-containing nano-silica composite particles are used as an active ingredient of surface treating agents, such as water- and oil-repellents.

EFFECT OF THE INVENTION

The fluorine-containing nano-silica composite particles according to the present invention not only have excellent water- and oil-repellency, but also can be stably dispersed in polar solvents, such as water, alcohol, and tetrahydrofuran. The fluorine-containing nano-silica composite particles also have excellent heat resistance at a high temperature (e.g., 800°C). Specifically, the increase in the composite particle diameter and the value of weight loss at a high temperature are reduced. Moreover, a perfluoroalkyl group having less than 8 carbon atoms do not lead to environmental pollution because they do not produce perfluorooctanoic acid and the like when released into the environment.

EMBODIMENTS FOR CARRYING OUT THE INVENTION

The fluorine-containing alcohol [I] is, for example, a polyfluoroalkyl alcohol represented by the general formula:

$$C_nF_{2n+1}(CH_2)_jOH \qquad [II]$$

n: 1 to 10, preferably 1 to 6
j: 1 to 6, preferably 2

The alkylene group A is, for example, a $-CH_2-$ group, $-CH_2CH_2-$ group, or the like. Examples of perfluoroalkyl alkyl alcohols having such an alkylene group include 2,2,2-trifluoroethanol ($CF_3CH_2OH$), 3,3,3-trifluoropropanol ($CF_3CH_2CH_2OH$), 2,2,3,3,3-pentafluoropropanol ($CF_3CF_2CH_2OH$), 3,3,4,4,4-pentafluorobutanol ($CF_3CF_2CH_2CH_2OH$), 2,2,3,3,4,4,5,5,5-nonafluoropentanol ($CF_3CF_2CF_2CH_2OH$), 3,3,4,4,5,5,6,6,6-nonafluorohexanol ($CF_3CF_2CF_2CF_2CH_2CH_2OH$), 3,3,4,4,5,5,6,6,7,7,8,8,8-tridecafluorooctanol ($CF_3CF_2CF_2CF_2CF_2CF_2CH_2CH_2OH$), and the like.

Moreover, a polyfluoroalkyl group refers to a group in which the terminal $-CF_3$ group of a perfluoroalkyl group is replaced by, for example, a $-CF_2H$ group. Examples thereof include 2,2,3,3-tetrafluoropropanol ($HCF_2CF_2CH_2OH$), 2,2,3,4,4,4-hexafluorobutanol ($CF_3CHFCF_2CH_2OH$), 2,2,3,3,4,4,5,5-octafluoropentanol ($HCF_2CF_2CF_2CF_2CH_2OH$), and the like.

The polyfluoroalkyl alcohol represented by the general formula [II] is described, for example, in Patent Document 2, and is synthesized through the following series of steps.

First, a polyfluoroalkyl iodide represented by the general formula:

$$C_nF_{2n+1}(CF_2CF_2)_b(CH_2CH_2)_cI$$

is reacted with N-methylformamide $HCONH(CH_3)$ to form a mixture of polyfluoroalkyl alcohol and its formate. Then, the mixture is hydrolyzed in the presence of an acid catalyst, thereby forming a polyfluoroalkyl alcohol of the formula:

$$C_nF_{2n+1}(CF_2CF_2)_b(CH_2CH_2)_cOH$$

Examples of the polyfluoroalkyl iodide include the following:

$$CF_3(CH_2CH_2)I$$

$$CF_3(CH_2CH_2)_2I$$

$$C_2F_5(CH_2CH_2)I$$

$$C_2F_5(CH_2CH_2)_2I$$

$$C_3F_7(CH_2CH_2)I$$

$$C_3F_7(CH_2CH_2)_2I$$

$$C_4F_9(CH_2CH_2)I$$

$$C_4F_9(CH_2CH_2)_2I$$

$$C_2F_5(CF_2CF_2)(CH_2CH_2)I$$

$$C_2F_5(CF_2CF_2)(CH_2CH_2)_2I$$

$$C_2F_5(CF_2CF_2)_2(CH_2CH_2)I$$

$$C_2F_5(CF_2CF_2)_2(CH_2CH_2)_2I$$

$$C_2F_5(CF_2CF_2)_3(CH_2CH_2)I$$

$$C_4F_9(CF_2CF_2)(CH_2CH_2)I$$

$$C_4F_9(CF_2CF_2)_2(CH_2CH_2)I$$

$$C_4F_9(CF_2CF_2)(CH_2CH_2)_2I$$

$$C_4F_9(CF_2CF_2)_2(CH_2CH_2)_2I$$

$$C_4F_9(CF_2CF_2)_3(CH_2CH_2)I$$

The fluorine-containing alcohol [I] may also be a fluorine-containing alcohol wherein the $R_F$ group is a polyfluoroalkyl group having 3 to 20 carbon atoms, preferably 6 to 10 carbon atoms, and A is an alkylene group having 2 to 6 carbon atoms, preferably 2 carbon atoms. Examples thereof include a polyfluoroalkyl alcohol represented by the general formula:

$$C_nF_{2n+1}(CH_2CF_2)_a(CF_2CF_2)_b(CH_2CH_2)_cOH \qquad [III]$$

n: 1 to 6, preferably 2 to 4
a: 1 to 4, preferably 1
b: 0 to 3, preferably 1 or 2
c: 1 to 3, preferably 1

The polyfluoroalkyl alcohol represented by the general formula [III] is disclosed in Patent Document 2, and synthesized through the following series of steps.
First of all, a polyfluoroalkyl iodide represented by the general formula:

$$C_nF_{2n+1}(CH_2CF_2)_a(CF_2CF_2)_b(CH_2CH_2)_cI$$

is reacted with N-methylformamide $HCONH(CH_3)$ to form a mixture of polyfluoroalkyl alcohol and its formate. The mixture is then subjected to a hydrolysis reaction in the presence of an acid catalyst to form a polyfluoroalkyl alcohol of the formula:

$$C_nF_{2n+1}(CH_2CF_2)_a(CF_2CF_2)_b(CH_2CH_2)_cOH$$

Examples of the polyfluoroalkyl iodide include the following:

$$CF_3(CH_2CF_2)(CH_2CH_2)I$$

$$C_2F_5(CH_2CF_2)(CH_2CH_2)I$$

$$C_2F_5(CH_2CF_2)(CH_2CH_2)_2I$$

$$C_3F_7(CH_2CF_2)(CH_2CH_2)I$$

$$C_3F_7(CH_2CF_2)(CH_2CH_2)_2I$$

$$C_4F_9(CH_2CF_2)(CH_2CH_2)I$$

$$C_4F_9(CH_2CF_2)(CH_2CH_2)_2I$$

$$C_2F_5(CH_2CF_2)(CF_2CF_2)(CH_2CH_2)I$$

$$C_2F_5(CH_2CF_2)(CF_2CF_2)(CH_2CH_2)_2I$$

$$C_2F_5(CH_2CF_2)_2(CF_2CF_2)(CH_2CH_2)I$$

$$C_2F_5(CH_2CF_2)_2(CF_2CF_2)(CH_2CH_2)_2I$$

$$C_4F_9(CH_2CF_2)(CF_2CF_2)(CH_2CH_2)I$$

$$C_4F_9(CH_2CF_2)_2(CF_2CF_2)(CH_2CH_2)I$$

$$C_4F_9(CH_2CF_2)(CF_2CF_2)(CH_2CH_2)_2I$$

$$C_4F_9(CH_2CF_2)_2(CF_2CF_2)(CH_2CH_2)_2I$$

The fluorine-containing alcohol [Ia] is, for example, a fluorine-containing alcohol wherein the $R_F'$ group is a perfluoroalkyl group having 3 to 305 carbon atoms, preferably 8 to 35 carbon atoms, and A is an alkylene group having 1 to 3 carbon atoms, preferably 1 carbon atom. Examples thereof include a hexafluoropropene oxide oligomer alcohol represented by the general formula:

$$C_mF_{2m+1}O[CF(CF_3)CF_2O]_dCF(CF_3)(CH_2)_eOH \quad [IIa]$$

m: 1 to 3, preferably 1
d: 0 to 100, preferably 2 to 50
e: 1 to 3, preferably 1

Moreover, the fluorine-containing alcohol [Ib] may be a fluorine-containing alcohol wherein the $R_F''$ group is a perfluoroalkylene group having 5 to 160 carbon atoms, and A is an alkylene group having 1 to 3 carbon atoms, preferably 1 carbon atom. Examples thereof include a perfluoroalkylene ether diol represented by the general formula:

$$HO(CH_2)_fCF(CF_3)[OCF_2CF(CF_3)]_gO(CF_2)_hO[CF(CF_3)CF_2O]_iCF(CF_3)(CH_2)_fOH \qquad [IIb]$$

f: 1 to 3, preferably 1
g+i: 0 to 50, preferably 2 to 50
h: 1 to 6, preferably 2

Among hexafluoropropene oxide oligomer alcohols represented by the general formula [IIa], a compound wherein m is 1 and e is 1 is described in Patent Document 3, and synthesized through the following step.
A fluorine-containing ether carboxylic acid alkyl ester represented by the general formula:

$CF_3O[CF(CF_3)CF_2O]_nCF(CF_3)COOR$ (R: an alkyl group, n: an integer of 0 to 12) is subjected to a reduction reaction using a reducing agent, such as sodium boron hydride.

Moreover, a perfluoroalkylene ether diol represented by the general formula [IIb] wherein f = 1 is disclosed in Patent Documents 4 and 5, and synthesized via the following series of steps:

$$FOCRfCOF \rightarrow H_3COOCRfCOOCH_3 \rightarrow HOCH_2RfCH_2OH$$

Rf: $-C(CF_3)[OCF_2C(CF_3)]_aO(CF_2)_cO[CF(CF_3)CF_2O]_bCF(CF_3)-$

Such a fluorine-containing alcohol and an alkoxysilane are reacted in the presence of an alkaline or acidic catalyst, thereby forming fluorine-containing nano composite particles.

The alkoxysilane is represented by the general formula:

$$(R_1O)_pSi(OR_2)_q(R_3)_r \qquad [IV]$$

$R_1$, $R_3$ : H, $C_1$-$C_6$ alkyl group, or aryl group
$R_2$: $C_1$-$C_6$ alkyl group or aryl group,

with the proviso that not all of $R_1$, $R_2$, and $R_3$ are aryl groups p + q + r: 4, with the proviso that q is not 0 examples thereof include trimethoxysilane, triethoxysilane, trimethoxymethylsilane, triethoxymethylsilane, trimethoxyphenylsilane, triethoxyphenylsilane, tetramethoxysilane, tetraethoxysilane, and the like.

The reaction between these components is performed in the presence of an alkaline or acid catalyst, such as aqueous ammonia, an aqueous solution of a hydroxide of an alkali metal or alkaline earth metal (e.g., sodium hydroxide, potassium hydroxide, or calcium hydroxide), hydrochloric acid, or sulfuric acid, at a temperature of about 0 to 100°C, preferably about 10 to 30°C, for about 0.5 to 48 hours, preferably about 1 to 10 hours.

The amount of fluorine-containing alcohol in the obtained fluorine-containing nano composite particles is about 1 to 50 mol%, preferably about 5 to 30 mol%. The composite particle size (measured by a dynamic light scattering method) is about 30 to 200 nm.

In the production of such a fluorine-containing nano composite, a condensation reaction with coexistence of organo nano-silica particles in the reaction system can produce fluorine-containing nano-silica composite particles in which a condensate comprising three components, i.e., a fluorine-containing alcohol, an alkoxysilane, and nano-silica particles, is formed.

As the nano-silica particles, organosilica sol having an average particle diameter (measured by a dynamic light scattering method) of 5 to 200 nm, preferably 10 to 100 nm, and having a primary particle diameter of 40 nm or less, preferably 5 to 30 nm, more preferably 10 to 20 nm, is used. Practically used are commercial products of Nissan Chemical Industries, Ltd., such as Methanol Silica Sol, Snowtex IPA-ST (isopropyl alcohol dispersion), Snowtex EG-ST (ethylene glycol dispersion), Snowtex MEK-ST (methyl ethyl ketone dispersion), and Snowtex MIBK-ST (methyl isobutyl ketone dispersion).

The ratio of these components is such that about 1 to 99 parts by weight, preferably about 10 to 50 parts by weight, of fluorine-containing alcohol, and about 1 to 99 parts by weight, preferably about 10 to 50 parts by weight, of alkoxysilane are used based on 100 parts by weight of the nano-silica particles. When the ratio of the fluorine-containing alcohol used is less than this range, water- and oil-repellency decreases. In contrast, when the ratio of the fluorine-containing alcohol used is greater than this range, dispersibility in solvents becomes poor. Moreover, when the ratio of alkoxysilane used is less than this range, dispersibility in solvents becomes poor. In contrast, when the ratio of alkoxysilane used is greater than this range, water- and oil-repellency decreases.

In the fluorine-containing nano-silica composite particles obtained as a reaction product, it is considered that the fluorine-containing alcohol is linked to a hydroxyl group on the surface of the nano-silica particles via a siloxane bond as a spacer. Therefore, the chemical and thermal stability of silica, and the excellent water- and oil-repellency, antifouling properties, and the like of fluorine are effectively exhibited. In fact, a glass surface treated with the fluorine-containing nano-silica composite particles exhibits excellent water- and oil-repellency, and also has the effect of, for example, reducing the weight loss at 800°C. Moreover, the particle size of the nano-silica composite particles, and the variation of the particle size show small values. The nano-silica composite particles are formed as a reaction product of a fluorine-containing alcohol, an alkoxysilane, and nano-silica particles; however, other components are allowed to be mixed as long as the object of the present invention is not impaired.

EXAMPLES

The following describes the present invention with reference to Examples.

Example 1

$CF_3(CF_2)_3(CH_2)_2OH$ [FA-4] (0.25 g) was added and dissolved in 30 ml of methanol. To the resulting solution, 1.67 g (0.50 g as nano-silica) of silica sol (Methanol Silica Sol, a product of Nissan Chemical Industries, Ltd.; nano-silica content: 30 wt.%, average particle diameter: 11 nm) and 0.25 ml of tetraethoxysilane (a product of Tokyo Chemical Industry Co., Ltd.; density: 0.93 g/ml) were added. While stirring the mixture with a magnetic stirrer, 0.25 ml of 25 wt.% aqueous ammonia was added, and the mixture was reacted for 5 hours.

After completion of the reaction, the methanol and aqueous ammonia were removed using an evaporator under reduced pressure, and the resulting powder was redispersed in approximately 20 ml of methanol overnight. The next day, centrifugation was performed using a centrifuge tube, the supernatant was removed, and fresh methanol was added to perform rinsing. After rinsing was performed 3 times, the opening of the centrifuge tube was covered with aluminum foil, and the tube was placed in an oven at 70°C overnight. The next day, the tube was placed and dried in a vacuum dryer at 50°C overnight, thereby obtaining 0.582 g (yield: 71%) of white powder.

The particle size of the obtained white powdery fluorine-containing nano-silica composite particles, and the variation of the particle size were measured in a methanol dispersion having a solid matters content of 1 g/L at 25°C by a dynamic light scattering (DLS) measurement method. Further, thermogravimetric analysis (TGA) was performed before calcining and after calcining up to 800°C. The heating rate in this case was 10°C/min. Moreover, the percentage of the weight loss due to calcining sintering with respect to the initial weight was also calculated.

Further, the dispersibility of the composite particles dispersed with a solid matters content of 1 wt.% in water [H_2O], methanol [MeOH], ethanol [EtOH], 1,2-dichloroethane [DCE], and tetrahydrofuran [THF] was visually observed, and the results were evaluated according to the following evaluation criteria.

○: Uniformly dispersed, transparent dispersion

Δ: Slightly dispersed, cloudy dispersion

× : Not dispersed, precipitated in dispersion medium

Examples 2 to 5

In Example 1, the amount of 25 wt.% aqueous ammonia was variously changed.

Examples 6 to 10

In Examples 1 to 5, the same amount (0.25g) of $CF_3(CF_2)_5(CH_2)_2OH$ [FA-6; $C_2F_5(CF_2CF_2)_2(CH_2CH_2)OH$] was used as the fluorine-containing alcohol.

Examples 11 to 15

In Examples 1 to 5, the same amount (0.25g) of $CF_3(CF_2)_7(CH_2)_2OH$ [FA-8; $C_2F_5(CF_2CF_2)_3(CH_2CH_2)OH$] was used as the fluorine-containing alcohol.

Table 1 below shows the amount of aqueous ammonia, recovered amount, yield, and various measurement results in the above Examples. Further, Table 2 shows the evaluation of dispersibility.

The yield was calculated by the following formula on the assumption that tetraalkoxysilane underwent a self-condensation reaction to form three-dimensional siloxane bonds Si-O and generate a -O-Si-O- [SiO_2] skeleton among them. When silica is not used, the yield is calculated based on C = 0.

$$\text{Yield (\%)} = A/[B + C + (D \times E \times F\,/\,G)] \times 100$$

A: weight of produced composite (g)
B: weight of fluorine-containing alcohol (g)
C: weight of silica (g)
D: volume of tetraalkoxysilane (ml)
E: density of tetraalkoxysilane (g/ml)
F: molar weight (g/mol) of $SiO_2$ derived from tetraalkoxysilane

G: molar weight (g/mol) of tetraalkoxysilane

Table 1

Fluorine-containing nano-silica composite particle size (nm)

| Ex. | aq. NH₃ (ml) | Recovery amount(g) | Yield (%) | Before calcining | After calcining up to 800°C | Weight loss(%) |
|---|---|---|---|---|---|---|
| 1 | 0.25 | 0.582 | 71 | 36.8± 9.9 | 39.0± 3.1 | 7 |
| 2 | 0.50 | 0.559 | 68 | 30.1± 7.3 | 39.5± 9.9 | 7 |
| 3 | 1.0 | 0.352 | 43 | 69.1±13.9 | 45.3±10.9 | 7 |
| 4 | 2.0 | 0.419 | 51 | 41.5±10.2 | 42.6± 9.2 | 7 |
| 5 | 4.0 | 0.571 | 70 | 34.0± 7.6 | 139.8±25.5 | 7 |
| 6 | 0.25 | 0.500 | 61 | 35.3± 8.3 | 53.3±11.4 | 8 |
| 7 | 0.50 | 0.580 | 71 | 40.5±11.3 | 40.5±12.0 | 6 |
| 8 | 1.0 | 0.590 | 72 | 40.5±13.0 | 62.3±18.5 | 6 |
| 9 | 2.0 | 0.488 | 60 | 105.3±19.0 | 97.5±30.2 | 7 |
| 10 | 4.0 | 0.426 | 52 | 45.4±13.2 | 60.9±17.1 | 6 |
| 11 | 0.25 | 0.521 | 64 | 41.7±13.7 | 81.7±21.6 | 7 |
| 12 | 0.50 | 0.481 | 59 | 28.2± 6.0 | 32.2± 9.8 | 6 |
| 13 | 1.0 | 0.475 | 58 | 56.6±11.5 | 53.7±10.2 | 6 |
| 14 | 2.0 | 0.516 | 63 | 53.6±11.4 | 55.1±14.5 | 6 |
| 15 | 4.0 | 0.565 | 69 | 39.7± 9.2 | 35.4±12.8 | 7 |

Table 2

| Ex. | H₂O | MeOH | EtOH | DCE | THF |
|---|---|---|---|---|---|
| 1 | Δ | ○ | ○ | ○ | ○ |
| 2 | ○ | ○ | ○ | ○ | ○ |
| 3 | ○ | ○ | ○ | ○ | ○ |
| 4 | ○ | ○ | ○ | ○ | ○ |
| 5 | ○ | ○ | ○ | ○ | ○ |
| 6 | ○ | ○ | ○ | ○ | ○ |
| 7 | ○ | ○ | ○ | ○ | ○ |
| 8 | Δ | ○ | ○ | ○ | ○ |
| 9 | ○ | ○ | ○ | ○ | ○ |
| 10 | ○ | ○ | ○ | ○ | ○ |
| 11 | ○ | ○ | ○ | ○ | ○ |
| 12 | ○ | ○ | ○ | ○ | ○ |
| 13 | ○ | ○ | ○ | ○ | ○ |
| 14 | ○ | ○ | ○ | ○ | ○ |
| 15 | ○ | ○ | ○ | ○ | ○ |

Reference Examples 1 to 3

In Examples 13 to 15, methanol silica sol was not used.

Table 3 below shows the amount of aqueous ammonia, produced fluorine-containing nano composite particle, recovered amount, yield, and various measurement results in the above Reference Examples 1 to 3. Further, Table 4 shows the evaluation of dispersibility.

Table 3

Fluorine-containing nano composite particle size (nm)

| Reference Ex. | aq. NH₃ (ml) | Recovery amount(g) | Yield (%) | Before calcining | After calcining up to 800°C | Weight loss(%) |
|---|---|---|---|---|---|---|
| 1 | 1.0 | 0.065 | 20 | 54.5±12.0 | 16.6± 3.8 | 18 |
| 2 | 2.0 | 0.059 | 19 | 21.1± 6.0 | 26.4± 6.0 | 17 |
| 3 | 4.0 | 0.065 | 20 | 37.3± 8.1 | 49.6±11.2 | 17 |

Table 4

| Reference Ex. | H₂O | MeOH | EtOH | DCE | THF |
|---|---|---|---|---|---|
| 1 | ○ | ○ | ○ | ○ | ○ |
| 2 | ○ | ○ | ○ | ○ | ○ |
| 3 | Δ | ○ | ○ | ○ | ○ |

Examples 21 to 35, and Reference Examples 11 to 13

Prepared glass slides were dipped in methanol dispersions (particle concentration: 5 g/L) of the fluorine-containing nano-silica composite particles before calcining (Examples 21 to 35) and the fluorine-containing nano composite particles before calcining (Reference Examples 11 to 13) obtained in Examples 1 to 15 and Reference Examples 1 to 3, and then dried at room temperature. Droplets (4 μl) were gently brought into contact with the obtained thin layer surfaces at room temperature, and the contact angle (unit: °) of the droplets adhering to n-dodecane or water was measured by the θ/2 method using a contact angle meter (Drop Master 300, produced by Kyowa Interface Science Co., Ltd.). The contact angle with water was measured with time. Table 5 below shows the obtained results.

Table 5

| Example | Composite | Dodecane | Water (elapsed time: min.) | | | | | | |
|---|---|---|---|---|---|---|---|---|---|
| | | | 0 | 5 | 10 | 15 | 20 | 25 | 30 |
| Ex.21 | Ex. 1 | 35 | 21 | 10 | 0 | 0 | 0 | 0 | 0 |
| Ex.22 | Ex. 2 | 7 | 22 | 17 | 15 | 13 | 11 | 10 | 7 |
| Ex.23 | Ex. 3 | 4 | 7 | 0 | 0 | 0 | 0 | 0 | 0 |
| Ex.24 | Ex. 4 | 0 | 7 | 0 | 0 | 0 | 0 | 0 | 0 |
| Ex.25 | Ex. 5 | 0 | 0 | 0 | 0 | 0 | 0 | 0 | 0 |
| Ex.26 | Ex. 6 | 49 | 28 | 0 | 0 | 0 | 0 | 0 | 0 |
| Ex.27 | Ex. 7 | 46 | 48 | 27 | 26 | 24 | 20 | 20 | 17 |
| Ex.28 | Ex. 8 | 16 | 19 | 19 | 16 | 14 | 13 | 10 | 8 |
| Ex.29 | Ex. 9 | 14 | 11 | 0 | 0 | 0 | 0 | 0 | 0 |
| Ex.30 | Ex. 10 | 19 | 16 | 7 | 0 | 0 | 0 | 0 | 0 |
| Ex.31 | Ex. 11 | 114 | 37 | 34 | 33 | 31 | 30 | 30 | 28 |
| Ex.32 | Ex. 12 | 108 | 61 | 58 | 52 | 49 | 46 | 45 | 41 |
| Ex.33 | Ex. 13 | 123 | 59 | 17 | 0 | 0 | 0 | 0 | 0 |
| Ex.34 | Ex. 14 | 125 | 23 | 13 | 10 | 8 | 0 | 0 | 0 |
| Ex.35 | Ex. 15 | 127 | 91 | 18 | 4 | 0 | 0 | 0 | 0 |
| Ref. Ex.11 | Ref. Ex. 1 | 50 | 52 | 48 | 45 | 38 | 34 | 29 | 25 |
| Ref. Ex.12 | Ref. Ex. 2 | 45 | 62 | 54 | 47 | 44 | 37 | 33 | 25 |
| Ref. Ex.13 | Ref. Ex. 3 | 43 | 35 | 29 | 26 | 22 | 18 | 14 | 9 |

Examples 41 to 45

In Examples 1 to 5, the same amount (0.25g) of $CF_3(CF_2)_3CH_2(CF_2)_5(CH_2)_2OH$ [DTFA; $C_4F_9(CH_2CF_2)(CF_2CF_2)_2(CH_2CH_2)OH$] was used as the fluorine-containing alcohol.

Table 6 below shows the amount of aqueous ammonia, recovered amount, yield, and various measurement results in the above Examples 41 to 45. Further, Table 7 shows the evaluation of dispersibility.

Table 6

| | | | | Fluorine-containing nano-silica composite particle size (nm) | | |
|---|---|---|---|---|---|---|
| Ex. | aq. NH$_3$ (ml) | Recovery amount(g) | Yield (%) | Before calcining | After calcining up to 800°C | Weight loss(%) |
| 41 | 0.25 | 0.580 | 71 | 54.5±19.3 | 71.9±15.3 | 7 |
| 42 | 0.50 | 0.604 | 74 | 44.3±13.8 | 46.2±10.4 | 7 |
| 43 | 1.0 | 0.523 | 64 | 55.6±12.3 | 53.1±14.7 | 8 |
| 44 | 2.0 | 0.504 | 62 | 53.6±10.3 | 54.0±12.9 | 6 |
| 45 | 4.0 | 0.578 | 71 | 63.6± 14.1 | 72.0±15.5 | 6 |

Table 7

| Ex. | H$_2$O | MeOH | EtOH | DCE | THF |
|---|---|---|---|---|---|
| 41 | ○ | ○ | ○ | ○ | ○ |
| 42 | ○ | ○ | ○ | ○ | ○ |
| 43 | ○ | ○ | ○ | ○ | ○ |
| 44 | ○ | ○ | ○ | ○ | ○ |
| 45 | ○ | ○ | ○ | ○ | ○ |

Reference Examples 21 to 23

In Examples 43 to 45, methanol silica sol was not used.

Table 8 below shows the amount of aqueous ammonia, produced fluorine-containing nano composite particle, recovered amount, yield, and various measurement results in the above Reference Examples 21 to 23. Further, Table 9 shows the evaluation of dispersibility.

Table 8

| | | | | Fluorine-containing nano composite particle size (nm) | | |
|---|---|---|---|---|---|---|
| Reference Ex. | aq. NH$_3$ (ml) | Recovery amount(g) | Yield (%) | Before calcining | After calcining up to 800°C | Weight loss(%) |
| 21 | 1.0 | 0.072 | 23 | 41.0± 8.7 | 16.3± 3.9 | 17 |
| 22 | 2.0 | 0.044 | 14 | 47.5±10.1 | 24.4± 5.7 | 20 |
| 23 | 4.0 | 0.069 | 22 | 81.5±14.5 | 24.2± 5.6 | 25 |

Table 9

| Reference Ex. | H$_2$O | MeOH | EtOH | DCE | THF |
|---|---|---|---|---|---|
| 21 | ○ | ○ | ○ | ○ | ○ |
| 22 | ○ | ○ | ○ | ○ | ○ |
| 23 | ○ | ○ | ○ | ○ | ○ |

Examples 46 to 50

Using methanol dispersions (particle concentration: 5 g/L) of the fluorine-containing nano-silica composite particles before calcining obtained in Examples 41 to 45, the contact angle (unit: °) of the droplets adhering to n-dodecane or water was measured by the above mentioned method. The contact angle with water was measured with time. Table 10 below shows the obtained results.

Table 10

| Ex. | Composite | Dodecane | Water (elapsed time: min.) | | | | | | |
|---|---|---|---|---|---|---|---|---|---|
| | | | 0 | 5 | 10 | 15 | 20 | 25 | 30 |
| 46 | Ex.41 | 82 | 30 | 21 | 20 | 14 | 9 | 0 | - |
| 47 | Ex.42 | 71 | 64 | 61 | 58 | 56 | 55 | 52 | 52 |
| 48 | Ex.43 | 55 | 79 | 77 | 75 | 68 | 61 | 58 | 52 |
| 49 | Ex.44 | 80 | 95 | 75 | 63 | 57 | 50 | 47 | 42 |
| 50 | Ex.45 | 47 | 113 | 82 | 72 | 64 | 57 | 53 | 49 |

Examples 61 to 65

In Example 2, the same amount (0.25 g) of each of the following compounds was used as the fluorine-containing alcohol.

Ex.61 : $CF_3(CF_2)_2OCF(CF_3)CF_2OCF(CF_3)CH_2OH$[PO-3-OH]
Ex.62 : $CF_3(CF_2)_2O[CF(CF_3)CF_2O]_4CF(CF_3)CH_2OH$[PO-6-OH]
Ex.63 : $HOCH_2CF(CF_3)OCF_2CF(CF_3)OCF_2CF_2OCF(CF_3)CH_2OH$ [OXF3PO-OH]
Ex.64 : $HOCH_2CF(CF_3)[OCF_2CF(CF_3)]_nOCF_2CF_2O[CF(CF_3)CF_2O]_m$ -$CF(CF_3)CH_2OH$(n+m=6) [OXF8PO-OH]
Ex.65 : $HOCH_2CF(CF_3)[OCF_2CF(CF_3)]_nOCF_2CF_2O[CF(CF_3)CF_2O]_m$-$CF(CF_3)CH_2OH$(n+m=12) [OXF14PO-OH]

Table 11 below shows the amount of aqueous ammonia, recovered amount, yield, and various measurement results in the above Examples 61 to 65. Further, Table 12 shows the evaluation of dispersibility.

Table 11

Fluorine-containing nano-silica composite particle size (nm)

| Ex. | aq. NH$_3$ (ml) | Recovery amount(g) | Yield (%) | Before calcining | After calcining up to 800°C | Weight loss(%) |
|---|---|---|---|---|---|---|
| 61 | 0.5 | 0.556 | 68 | 42.2± 4.2 | 35.2± 8.4 | 5 |
| 62 | 0.5 | 0.580 | 71 | 130.5±27.5 | 29.8± 5.8 | 7 |
| 63 | 0.5 | 0.466 | 57 | 55.5± 7.9 | 23.7± 7.1 | 5 |
| 64 | 0.5 | 0.359 | 44 | 96.9± 10.9 | 30.0± 6.9 | 11 |
| 65 | 0.5 | 0.507 | 62 | 90.8± 14.9 | 90.8± 9.7 | 20 |

Table 12

| Ex. | H$_2$O | MeOH | EtOH | DCE | THF |
|---|---|---|---|---|---|
| 61 | ○ | ○ | ○ | ○ | ○ |
| 62 | ○ | ○ | ○ | ○ | ○ |
| 63 | ○ | ○ | ○ | ○ | ○ |
| 64 | ○ | ○ | ○ | ○ | ○ |
| 65 | ○ | ○ | ○ | ○ | ○ |

Reference Examples 31 to 33

In Example 61, methanol silica sol was not used, and the amount of 25% aqueous ammonia was variously changed.

Reference Examples 34 to 36

In Example 62, methanol silica sol was not used, and the amount of 25% aqueous ammonia was variously changed.

Reference Example 37

In Example 63, methanol silica sol was not used, and the amount of 25% aqueous ammonia was changed to 4.0ml.

Reference Example 38

In Example 64, methanol silica sol was not used, and the amount of 25% aqueous ammonia was changed to 4.0ml.

Reference Example 39

In Example 65, methanol silica sol was not used, and the amount of 25% aqueous ammonia was changed to 4.0ml.

Table 13 below shows the amount of aqueous ammonia, produced fluorine-containing nano composite particle, recovered amount, yield, and various measurement results in the above Reference Examples 31 to 39. Further, Table 14 shows the evaluation of dispersibility.

Table 13

| Reference Ex. | aq. NH$_3$ (ml) | Recovery amount(g) | Yield (%) | Fluorine-containing nano composite particle size (nm) | | Weight loss(%) |
| --- | --- | --- | --- | --- | --- | --- |
| | | | | Before calcining | After calcining up to 800°C | |
| 31 | 1.0 | 0.073 | 23 | 48.3± 4.8 | 34.2± 4.4 | 16 |
| 32 | 2.0 | 0.073 | 23 | 53.1± 5.1 | 38.0± 9.2 | 12 |
| 33 | 4.0 | 0.067 | 21 | 45.1± 6.5 | 51.1±16.8 | 12 |
| 34 | 1.0 | 0.070 | 22 | 141.5±31.8 | 26.6± 6.3 | 16 |
| 35 | 2.0 | 0.048 | 15 | 80.2± 31.2 | 80.5±21.2 | 13 |
| 36 | 4.0 | 0.063 | 20 | 69.2±10.1 | 69.4±12.5 | 12 |
| 37 | 4.0 | 0.051 | 16 | 60.5±12.4 | 55.1±12..1 | 12 |
| 38 | 4.0 | 0.063 | 20 | 55.7± 8.9 | 65.4±12.1 | 13 |
| 39 | 4.0 | 0.171 | 54 | 63.2± 6.7 | 53.5± 7.8 | - |

Table 14

| Reference Ex. | H$_2$O | MeOH | EtOH | DCE | THF |
| --- | --- | --- | --- | --- | --- |
| 31 | ○ | ○ | ○ | ○ | ○ |
| 32 | ○ | ○ | ○ | ○ | ○ |
| 33 | ○ | ○ | ○ | ○ | ○ |
| 34 | ○ | ○ | ○ | ○ | ○ |
| 35 | Δ | ○ | ○ | ○ | ○ |
| 36 | ○ | ○ | ○ | ○ | ○ |
| 37 | ○ | ○ | ○ | ○ | ○ |
| 38 | Δ | ○ | ○ | ○ | ○ |

(continued)

| Reference Ex. | H₂O | MeOH | EtOH | DCE | THF |
|---|---|---|---|---|---|
| 39 | ○ | ○ | ○ | ○ | ○ |

Examples 66 to 70

Using the methanol dispersions (particle concentration: 5 g/L) of the fluorine-containing nano-silica composite particles before calcining obtained in above Examples 61 to 65, the contact angle (unit: °) of the droplets adhering to n-dodecane or water was measured by the above mentioned method. The contact angle with water was measured with time. Table 15 below shows the obtained results.

Table 15

| Ex. | Composite | Dodecane | Water (elapsed time: min.) | | | | | | |
|---|---|---|---|---|---|---|---|---|---|
| | | | 0 | 5 | 10 | 15 | 20 | 25 | 30 |
| 66 | Ex.61 | 72 | 23 | 0 | 0 | 0 | 0 | 0 | 0 |
| 67 | Ex.62 | 62 | 0 | 0 | 0 | 0 | 0 | 0 | 0 |
| 68 | Ex.63 | 49 | 18 | 0 | 0 | 0 | 0 | 0 | 0 |
| 69 | Ex.64 | 51 | 24 | 11 | 0 | 0 | 0 | 0 | 0 |
| 70 | Ex.65 | 66 | 17 | 0 | 0 | 0 | 0 | 0 | 0 |

## Claims

1.  Fluorine-containing nano-silica composite particles comprising a condensate of a fluorine-containing alcohol represented by the general formula:

$$R_F\text{-A-OH} \qquad [I]$$

wherein $R_F$ is a perfluoroalkyl group or a polyfluoroalkyl group in which some of the fluorine atoms of the perfluoroalkyl group are replaced by hydrogen atoms, and A is an alkylene group having 1 to 6 carbon atoms; and an alkoxysilane with nano-silica particles.

2.  The fluorine-containing nano-silica composite particles according to claim 1, wherein the fluorine-containing alcohol represented by the general formula [I] is a polyfluoroalkyl alcohol represented by the general formula:

$$C_nF_{2n+1}(CH_2)_jOH \qquad [II]$$

wherein n is an integer of 1 to 10, and j is an integer of 1 to 6.

3.  The fluorine-containing nano-silica composite particles according to claim 1, wherein the fluorine-containing alcohol represented by the general formula [I] is a polyfluoroalkyl alcohol represented by the general formula:

$$C_nF_{2n+1}(CH_2CF_2)_a(CF_2CF_2)_b(CH_2CH_2)_cOH \qquad [III]$$

wherein n is an integer of 1 to 6, a is an integer of 1 to 4, b is an integer of 0 to 3, and c is an integer of 1 to 3.

4.  The fluorine-containing nano-silica composite particles according to claim 1, wherein the alkoxysilane is a silane derivative represented by the general formula:

$$(R_1O)_pSi(OR_2)_q(R_3)_r \qquad [IV]$$

wherein $R_1$ and $R_3$ are each a hydrogen atom, an alkyl group having 1 to 6 carbon atoms, or an aryl group; $R_2$ is an alkyl group having 1 to 6 carbon atoms or an aryl group, with the proviso that not all of $R_1$, $R_2$, and $R_3$ are aryl groups; and p + q + r is 4, with the proviso that q is not 0.

5. A method for producing fluorine-containing nano-silica composite particles, the method comprising subjecting the fluorine-containing alcohol [I] according to claim 1 and an alkoxysilane to a condensation reaction in the presence of nano-silica particles using an alkaline or acidic catalyst.

6. Fluorine-containing nano-silica composite particles comprising a condensate of a fluorine-containing alcohol represented by the general formula:

$$R_F'\text{-A-OH} \qquad [Ia]$$

or the general formula:

$$HO\text{-A-}R_F''\text{-A-OH} \qquad [Ib]$$

wherein $R_F'$ is a liner or branched perfluoroalkyl group containing an O, S, or N atom, $R_F''$ is a linear or branched perfluoroalkylene group containing an O, S, or N atom, and A is an alkylene group having 1 to 6 carbon atoms; and an alkoxysilane with nano-silica particles.

7. The fluorine-containing nano-silica composite particles according to claim 6, wherein the fluorine-containing alcohol represented by the general formula [Ia] is a hexafluoropropene oxide oligomer alcohol represented by the general formula:

$$C_mF_{2m+1}O[CF(CF_3)CF_2O]_dCF(CF_3)(CH_2)_cOH \qquad [IIa]$$

wherein m is an integer of 1 to 3, d is an integer of 0 to 100, and e is an integer of 1 to 3.

8. The fluorine-containing nano-silica composite particles according to claim 6, wherein the fluorine-containing alcohol represented by the general formula [Ib] is a perfluoroalkylene ether diol represented by the general formula:

$$HO(CH_2)_fCF(CF_3)[OCF_2CF(CF_3)]_gO(CF_2)hO[CF(CF_3)CF_2O]_iCF(CF_3)(CH_2)_tOH \qquad [IIb]$$

wherein f is an integer of 1 to 3, g + i is an integer of 0 to 50, and h is an integer of 1 to 6.

9. The fluorine-containing nano-silica composite particles according to claim 6, wherein the alkoxysilane is a silane derivative represented by the general formula:

$$(R_1O)_pSi(OR_2)_q(R_3)_r \qquad [III]$$

wherein $R_1$ and $R_3$ are each a hydrogen atom, an alkyl group having 1 to 6 carbon atoms, or an aryl group; $R_2$ is an alkyl group having 1 to 6 carbon atoms or an aryl group, with the proviso that not all of $R_1$, $R_2$, and $R_3$ are aryl groups; and p + q + r is 4, with the proviso that q is not 0.

10. A method for producing fluorine-containing nano-silica composite particles, the method comprising subjecting the fluorine-containing alcohol [Ia] or [Ib] according to claim 6 and an alkoxysilane to a condensation reaction in the presence of nano-silica particles using an alkaline or acidic catalyst.

## INTERNATIONAL SEARCH REPORT

International application No.

PCT/JP2014/055818

A. CLASSIFICATION OF SUBJECT MATTER

*C08G77/24*(2006.01)i, *B82Y30/00*(2011.01)i, *B82Y40/00*(2011.01)i, *C07C31/38* (2006.01)i, *C07F7/04*(2006.01)i, *C08G77/18*(2006.01)i

According to International Patent Classification (IPC) or to both national classification and IPC

B. FIELDS SEARCHED

Minimum documentation searched (classification system followed by classification symbols)
C08G77/24, B82Y30/00, B82Y40/00, C07C31/38, C07F7/04, C08G77/18, C09D1/00-201/00

Documentation searched other than minimum documentation to the extent that such documents are included in the fields searched
Jitsuyo Shinan Koho         1922-1996   Jitsuyo Shinan Toroku Koho   1996-2014
Kokai Jitsuyo Shinan Koho   1971-2014   Toroku Jitsuyo Shinan Koho   1994-2014

Electronic data base consulted during the international search (name of data base and, where practicable, search terms used)
JSTPlus(JDreamIII), JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 2004-285111 A (Arakawa Chemical Industries, Ltd.), 14 October 2004 (14.10.2004), entire text (Family: none) | 1-10 |
| A | JP 5-186719 A (Hitachi Chemical Co., Ltd.), 27 July 1993 (27.07.1993), entire text (Family: none) | 1-10 |
| A | JP 2004-244428 A (Arakawa Chemical Industries, Ltd.), 02 September 2004 (02.09.2004), entire text (Family: none) | 1-10 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 May, 2014 (13.05.14) | 20 May, 2014 (20.05.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

International application No.

PCT/JP2014/055818

**INTERNATIONAL SEARCH REPORT**

C (Continuation). DOCUMENTS CONSIDERED TO BE RELEVANT

| Category* | Citation of document, with indication, where appropriate, of the relevant passages | Relevant to claim No. |
|---|---|---|
| A | WO 2007/105633 A1 (Unimatec Co., Ltd.),<br>20 September 2007 (20.09.2007),<br>entire text<br>& JP 4674604 B          & US 2009/0036706 A1<br>& EP 1995228 A1         & DE 602007014332 D<br>& CA 2643067 A          & CN 101400633 A | 1-10 |
| A | JP 2002-514260 A (Ameron International Corp.),<br>14 May 2002 (14.05.2002),<br>entire text<br>& US 6013752 A          & EP 1716827 A2 | 1-10 |
| A | JP 2008-514744 A (Evonik Degussa GmbH),<br>08 May 2008 (08.05.2008),<br>entire text<br>& EP 1812498 A2         & WO 2006/032512 A2<br>& DE 102004046385 A     & KR 10-2007-0059172 A<br>& CN 101052667 A | 1-10 |
| A | JP 61-103930 A (Dow Corning Corp.),<br>22 May 1986 (22.05.1986),<br>entire text<br>& US 4554296 A          & EP 179598 A2<br>& DE 3578631 D          & CA 1246286 A | 1-10 |
| A | JP 2006-143731 A (Clariant Produkte<br>(Deutschland) GmbH),<br>08 June 2006 (08.06.2006),<br>entire text<br>& US 2006/0111581 A1    & EP 1659126 A1 | 1-10 |

Form PCT/ISA/210 (continuation of second sheet) (July 2009)

**EP 2 966 112 A1**

**REFERENCES CITED IN THE DESCRIPTION**

**Patent documents cited in the description**

- JP 2011511113 A
- JP 4674604 B
- WO 2007080949 A1
- JP 2008038015 A
- US 3574770 P